# EUROPEAN PATENT APPLICATION

(11) **EP 1 238 670 A1**
(43) Date of publication of application: **11.09.2002**
(21) Application number: 01401765.1
(22) Date of filing: 02.07.2001
(51) Int. Cl.: A61K 31/715, A61K 31/557, A61K 31/34, A61K 31/195, A61P 9/00

(54) **Composition and method for treating and preventing cardiovascular disease and hypertension.**

(30) Priority: 07.03.2001 US 255296 P
(71) Applicant: Daniels, Bruce, Oklahoma City, Oklahoma 73109 (US)
(72) Inventor: Daniels, Bruce, Oklahoma City, Oklahoma 73109 (US)
(74) Representative: Powell, Stephen David

(57) **Abstract**

The present invention is directed to a composition of material for treating a patient susceptible to or suffering from either congestive heart failure, stroke, vascular dysfunction, hypertension, smooth muscle cell hypertrophy, platelet disorder cerebrovascular ischemia, or peripheral vascular ischemia, said composition comprising a therapeutically effective amount of a first substance selected from the group comprising either heparin, heparin sulfate, heparan, taprostene, prostacyclin, or physiologically acceptable salts thereof and therapeutically effective amount of a second substance selected from a group comprising either arginine, L-arginine, arginine sulfate, or physiologically acceptable salts thereof.

## Description

### CROSS-REFERENCE TO RELATED APPLICATIONS:

U.S. PATENT # 6255296, ISSUE DATE: 07/03/01

STATEMENT REGARDING FEDERALLY-
SPONSORED RESEARCH: Not Applicable.

REFERENCE TO A MICROFICHE APPENDIX: Not Applicable.

### FIELD OF THE INVENTION

This invention relates generally to a composition of material and method for treating a patient susceptible to or suffering from cardiovascular disease, more particularly to preventing and treating congestive heart failure, smooth muscle cell hypertrophy, cardiac cell hypertrophy, vascular platelet accumulations, cerebrovascular ischemia, peripheral vascular ischemia, or hypertension.

### BACKGROUND OF THE INVENTION

Cardiovascular diseases, hypertension and their associated complications are a principal cause of disabilities and deaths of individuals in the United States and Western Europe. For example, in recent years more than 500,000 deaths have occurred annually in the United States alone as a result of coronary artery disease.

There has been an ongoing search for effective long-term treatment for diseases of the heart and arteries, such as those listed above. Prior treatments for such diseases include administration of vasodilators, angioplasty and by-pass surgery, for example. Such treatments have met with great disapproval due to risks versus benefits gained by the various treatments. Such treatments have serious shortcomings in long-term effectiveness. The use of vasodilator drugs and mechanical treatments for those diseases have to date been ineffective for favorable long-term results. The outcome with current treatments is minimally impacted because the treatments are directed toward effects of the underlying disease process rather than initial molecular cause of the disease.

For example, the rationale for vasoactive drugs is to reduce blood pressure by acting directly or indirectly on vascular and/or cardiac smooth muscle, thereby decreasing vascular resistance to flow. Such drugs do not treat initial cause of elevated pressure and abnormal flow. Rather, they seek to reduce the resulting effect of the disease or disorder. Such drugs activate the sympathetic nervous system by way of a baroreceptor reflex to produce an increased heart rate and force of myocardial contraction, which are not beneficial effects. Other side effects from such drugs include headache, heart palpitations, anxiety, mild depression, dry-mouth, unpleasant taste in the mouth, nausea, vomiting, angina, myocardial infarction, congestive heart failure, decreased cardiac output, fluid retention, fatigue, and weakness. Pharmacological treatment is not specific in its effect on the initial molecular cause of the disease activity, and treats a limited spectrum of effects in the diseases which are multifactorial.

As a further example, improved outcome in the listed diseases is seen with cholesterol reduction and drug treatment for lipid disorders. These treatments do not address or prevent cellular or molecular reactions attributed to platelets, macrophages, neutrophils, lymphocytes, smooth muscle cells, and other cell types known to be involved in those diseases,

Likewise, angioplasty and bypass surgery have been minimally successful long-term. Current mechanical and pharmacological treatments focus on a partial or completely occluded vessel where, at the particular site, it is either unclogged or bypassed with connecting vessels. These treatments fail to address physiologic derangements of homeostatic systems which allow the occlusive process to begin and progress. Accordingly, there is frequent recurrent occlusion in the initially treated vessel and no treatment for sites not judged to be adequately affected.

There remains a great need for treatment which prevents failure of homeostatic controls and which restores these controls once derangements begin to develop. Restoration of endogenous regulatory systems and cellular domains to a healthy state prevents occlusion, platelet development and hypertension.

### SUMMARY OF THE INVENTION

It is a conception of the inventor that a cellular matrix composed of heparin-L-arginine-water polymers is responsible for controlling molecular milieu comprising the human cellular environment. It is these polymers which determine the protein distribution, auto-immune functionality, DNA-RNA transcription regulation, and the physical properties of cells.

It is an object of the present invention to provide a composition and method of treating and preventing the listed diseases by co-administration of a therapeutically effective composition consisting of exogenous L-arginine and exogenous heparin or their respective analogs, in which components of the endothelium and its contiguous tissues are stimulated to therapeutically prevent and minimize adverse molecular reactions associated with those diseases by sustained production of endogenous nitric oxide and heparin.

### DETAILED DESCRIPTION OF THE PREFERRED EMBODIMENT

The present invention is directed to a composition and method for inhibiting the aforesaid listed diseases in a patient. In accordance with the invention, a patient susceptible to or suffering from a disease such as congestive heart failure, smooth muscle cell hypertrophy, cardiac cell hypertrophy, vascular platelet aggregation, cerebrovascular ischemia, peripheral vascular ischemia, or hypertension, is treated by co-administration of a therapeutically effective amount of a first substance characterized as exogenous non-anticoagulant heparin, also known as heparan, or its functional analogs, and a second substance characterized as exogenous L-arginine or its functional analogs.

A therapeutically effective amount of that heparin is defined primarily by clinical response in a patient, and ranges from about 2,000 IU to 200,000 IU daily, on variable schedule.

For example, the heparin is characterized such that it should be an amount sufficient to produce a sustained production cycle of endogenous nitric oxide.

A therapeutically effective amount of L-arginine ranges from 500 mg to 50,000 mg daily dependent on the underlying condition and nature of the physiological processes requiring treatment. For example, the L-arginine should be a sufficient amount (1) to continually produce a therapeutically-effective level of endogenous nitric oxide, (2) to increase prostacyclin secretion within the vascular system and its contiguous tissues, (3) to reduce intrusion of extra-cellular proteins and heparin-binding proteins within that system, and (4) to increase extra-cellular matrix barrier properties which in turn decrease extra-cellular matrix pore size and permeability. The inventive method necessarily includes the steps of monitoring the patient in order to ascertain if therapeutically-effective levels of nitric oxide are produced on a sustained basis.

Again, effective doses of heparin vary with the particular patient condition and the method of administration. For example, it is noticed that subcutaneous injection of heparin results in greater concentration in the cellular and membrane domains than by intravenous injection.

L-arginine is added together with or separately from the heparin.

The physiological condition of the patient will largely dictate the required dosages and frequencies of L-arginine administration, i.e. weight, age, disease, sex.

The composition and method can be effected by oral, sublingual, subcutaneous, intravenous, transdermal or rectal administrations in admixture with pharmaceutical excipients or vehicles including implantation or controlled-release devices.

For example, the compound of heparan and L-arginine can be dispersed in a physiologically acceptable, non-toxic liquid vehicle, such as water. Alternatively, the compound can be given in tablet, capsule, powder, granules or coated tablet form. The compound can be made in a conventional manner, and may be mixed with conventional pharmaceutical auxiliaries, such as binders, fillers, preservatives, tablet disintegrators, flow regulators, plasticizers, wetting agents, dispersants, emulsifiers, solvents, retarding agents and/or anti-oxidants. It also can be contained or complexed with lipids in various formulations and molecular arrangements, e.g. lipid tubules.

An efficiently operating homeostatic system is crucial to cellular function within mammalian organisms. In a healthy state, there is formed a gel matrix of heparin, arginine and water polymers, which houses a plurality of other molecules by accommodating dynamic binding of and release of such molecules without reaching concentration levels which destroy the gel structure and its regulatory functionalities.

Commercially, heparin or heparan is normally derived from animal tissue such as livers and lungs of cattle, bovine species and sheep.

Heparin and heparin-like compounds such as heparan have also been found in plant tissue where the heparin or heparin-like compound is bound to the plant proteins in the form of a complex. Heparin and heparin-like compound derived from plant tissue are of particular importance because they are considerably less expensive than heparin and heparin-like compounds harvested from animal tissue.

Plants which contain heparin or heparin-like compounds will provide a suitable source for the present invention. Typical plant sources of heparin or heparin-like compounds include artemisia princeps, nothogenia fastigia (red seaweed), copallina pililifera (red algas), cladophora sacrilis (green seaweed), chaotomorpha anteninna (green seaweed), aopallina officinalis (red seaweed), monostrom nitidum, laminaria japonica, filipendula ulmaria (meadowsweet), ecklonia kuroma (brown seaweed), ascophyllum nodosum (brown seaweed), ginkgo biloba, ulva rigida (green algae), stichopus japonicus (seacucumber), panax ginseng, spiralina maxima, spirulina platensis, laurencia gemmifera (red seaweed), larix (larchwood), and analogs thereof.

Such plants are considered to be an effective and efficient source of heparin or heparin-like compounds for use in the present invention.

Polymer strands are an organizing determinant for membranes, proteins, receptors, ion channels, cell organelles, nuclear membranes, membrane pores, and other complex cellular constituents. The polymers organize water into arenas for confining bilipid layer membranes.

Heparin's high sulfate content imparts a high negative charge which attracts and binds positively charged substances like basic amino acids, basic domains of proteins and peptides, cations, water and other such charged molecules.

Arginine has a high positive charge and strongly associates with heparin along membrane surfaces such as the endothelium, its contiguous tissues, and in association with water, organize as gel matrix.

The gel may be in a constant state of change, including transitions from one state or phase to another. As such, conformation can change and derangements occur as different substances move in and out of the gel changing gel properties,

A healthy gel matrix is continually formed and maintained from endogenous heparin, endogenous L-arginine and water.

The healthy gel structure has a conformation that preferentially neutralizes and isolates those foreign molecules which precipitate onset of the aforesaid diseases. The capacity to neutralize and isolate intrusions of such molecules before the gel structure collapses and loses its functionality is an important characteristic of the gel system produced and maintained by this inventive method.

As these intrusions accumulate locally or in a distributed fashion, they cause an interference within the gellular association of heparin and L-arginine. The interference can cause the gel structure to deteriorate, thus increasing its porosity or collapse altogether in a localized or distributed fashion. In addition, the intrusion may trigger a release of other bound polar molecules, such as calcium which would induce a non-homeostatic event.

The permeability created by the intrusion of such adverse molecules allows macromolecules or cells to compromise gel integrity.

These intrusions result in a displacement of arginine and decreased generation of nitric oxide as an additional effect. Intrusions limit the binding capacity of the heparin for L-arginine within the gel.

In order to reverse this disruption of the gel matrix caused by removal of arginine and/or heparin, the present invention employs the composition in order to maintain and rejuvenate the gel matrix and its functionality. In this regard, the present invention utilizes a full range of low molecular weight heparin and L-arginine to give optimal pore closure and stabilization to the gel matrix and its molecular structure.

The steps of heparan and L-arginine co-administration also lead to reduced plaque accumulation.

The expression of the endogenous heparin is surface receptor dependent upon the endothelium and its contiguous tissues in that the prostacyclin, in association with heparin at the endothelial and gel surfaces, generates a sustained signal to the golgi apparatus resulting in sustained and continual production of endogenous heparin and endogenous nitric oxide. Added exogenous heparin accumulates at the blood/endothelium surface thereby reconstituting the prostacyclin receptors which may have been damaged and depleted over time. Nitric oxide production at or near the same surface occurs from nitric oxide synthase action on exogenous and endogenous L-arginine substrate. This nitric oxide amplifies the signal by increasing the local concentration of prostacyclin, whose production is mediated by the nitric oxide.

Thus, heparin is generated in quantities sufficient to cause an unexpected level of reassociation of L-arginine and heparin resulting in restoration and protection of the endothelium and gel structure, as well as neutralizing and isolating potentially injurious molecules in the gel matrix.

Addition of heparin to the gel system protects the functionality of the L-arginine in the gel, and addition of L-arginine to the gel system protects the functionality of heparin in the gel. In the extragellular medium, the ability of heparin to bind and quiesce molecules is augmented by simultaneous addition of exogenous heparin and exogenous L-arginine, wherein exogenous heparin is binding to extragellular potentially-intruding molecules, thus allowing existing gellular heparin to associate with gellular L-arginine. Exogenous L-arginine now becomes the more available substrate for nitric oxide synthase, thereby protecting gellular endogenous L-arginine from the nitric oxide synthase activity and allowing the endogenous L-arginine to continuously re-associate with the gellular heparin, thus protecting and restoring gel and endothelial functionality.

Nitric oxide produced from L-arginine is an important physiological mediator. The enzyme responsible for nitric oxide production, nitric oxide synthase, requires CA⁺⁺ and Calmodulin. The functionality of the heparin-arginine gel includes its binding and regulation of CA⁺⁺ and Calmodulin. By regulating Calmodulin activity, the heparin-arginine gel regulates nitric oxide synthase activity responsible for nitric oxide production.

The binding of water, small anions and cations within the heparin-L-arginine-water gel is facilitated by pi-bonding properties inherent in the saccharide ring structure within the heparin polymers. Changes in the shared electron density and electrical charge variation regulate the state of solvation and conformation of the gel polymers. Thus, small anion and cation binding induces changes in the state of solvation, changes in catalytic and hydrolytic properties of water, and changes in capacity of the gel to bind water and other molecules.

Low to high molecular weight heparin preferably having a high degree of sulfation, can be used as well as standard heparin as is commercially available. Human, animal, and recombinant heparin sources are believed to be useful in practicing the invention and are capable of stimulating the full range of responses claimed herein. The source of exogenous heparin, including the possibility of human recombinant heparin, and the source of L-arginine impart no special or additional properties to the homeostatic functionalities observed for those individual elements or their conjoined, synergistic functionalities. Various glycosaminoglycans, similar to heparin, are subject to in vivo epimerization and sulfation resulting from agents which promote acylation reactions and sulfation reactions, such as acetyl salicylic acid, thereby producing heparin or heparin functionality. Thus, for example, heparan sulfate is considered an analog of heparin. Heparin can be used in the form of its salts with physiologically tolerated bases, for example, sodium, calcium, magnesium, diethylamine, triethylamine or triethanolamine. Promoters of increased heparin production, such as prostacyclin, are the functional equivalent of heparin, as would be analog's thereof, such as taprostene and may be employed in the present method.

Endothelial cell injury occurs from free radicals. Heparin binds super oxide dismutase which absorbs high energy electrons and deactivates free radicals, Heparin and nitric oxide bind free radicals thereby preventing damage to endothelial cells.

Congestive heart failure is in part due to free radical injury to cardiovascular tissues. Heparin, super oxide dismutase and nitric oxide all attack and neutralize free radicals. Thus, the diseases which are precipitated by cellular injury from free radicals, are effectively neutralized and prevented by the present method. Also, heparin aids in the reconstruction of damaged tissue by complexing and removing extracellular matrix protein accumulations, e.g. fibronectin with consequent reverse of organ hypertrophy states.

Heparin, via its association with L-arginine, enhances unexpected levels of regeneration of the endothelium following injury to it and its contiguous tissues.

It will be readily apparent to those skilled in the art that many modifications, derivations and improvements are within the scope of the invention. Such modifications, derivations, and improvements should be accorded full scope of protection by the claims appended hereto.

## Claims

1. A composition of material for treating a patient susceptible to or suffering from either congestive heart failure, stroke, vascular dysfunction, hypertension, smooth muscle cell hypertrophy, platelet disorder cerebrovascular ischemia, or peripheral vascular ischemia, said composition comprising a therapeutically effective amount of a first substance selected from the group comprising either heparin, heparin sulfate, heparan, taprostene, prostacyclin, or physiologically acceptable salts thereof and therapeutically effective amount of a second substance selected from a group comprising either arginine, L-arginine, arginine sulfate, or physiologically acceptable salts thereof.

2. A composition of material according to claim 1, wherein said composition is mixed with conventional pharmaceutical auxiliaries.

3. A composition of material according to any of claims 1 or 2, wherein said conventional pharmaceutical auxiliaries are selected from binders, fillers, preservatives, tablet disintegrators, flow regulators, plasticizers, wetting agents, dispersants, emulsifiers, solvents, solvents, retarding agents and anti-oxydants.

4. A composition of material according to any of claims 1 or 3, wherein said therapeutically effective amount of a first substance selected from the group comprising either heparin, heparin sulfate, heparan, taprostene, prostacyclin, or physiologically acceptable salts thereof ranges from about 2,000 to 200,000 IU daily.

5. A composition of material according to any of claims 1 to 4, wherein said therapeutically effective amount of a second substance selected from a group comprising either arginine, L-arginine, arginine sulfate, or physiologically acceptable salts thereof ranges from about 500 mg to 50,000 mg daily.

6. A composition of material according to any of claims 1 to 5, wherein said first substance is exogenous heparan and said second substance is exogenous L-arginine.

7. A composition of material according to any of claims 1 to 6, wherein said composition is contained or complexed with lipids in various formulations and molecular arrangements.

8. A composition of material according to any of claims 1 to 7, wherein said composition is administered either orally, sublingually, subcutaneously, intravenously, rectally, transdermally or intrapulmonary.

9. A composition of material according to any of claims 1 to 8, wherein said composition is dispersed in a physiologically acceptable, non-toxic liquid vehicule.

10. A composition of material according to any of claims 1 to 8, wherein said composition is given in tablet, capsule, powder, granules or coated tablet form.
